# EUROPEAN PATENT APPLICATION

(11) **EP 0 658 531 A1**
(43) Date of publication of application: **21.06.1995**
(21) Application number: 94908814.0
(22) Date of filing: 09.08.1993
(51) Int. Cl.: C07C 19/08, C07C 17/00, B01J 23/40

(54) **PROCESS FOR PRODUCING 1,1,1,2,3-PENTAFLUOROPROPANE**

(30) Priority: 04.09.1992 JP 262864/92
(71) Applicant: DAIKIN INDUSTRIES, LIMITED, Osaka-shi, Osaka-fu 530 (JP)
(72) Inventor: NAKADA, Tatsuo, Yodogawa Works of, Settsu-shi, Osaka 566 (JP); AOYAMA, Hirokazu, Yodogawa Works of, Settsu-shi, Osaka 566 (JP)
(74) Representative: TER MEER - MÜLLER - STEINMEISTER & PARTNER
(86) International application number: JP9301118
(87) International publication number: WO9405612

(57) **Abstract**

A process for producing 1,1,1,2,3-pentafluoropropane which comprises hydrogenating 1,2,3,3,3-pentafluoro-1-chloropropene with hydrogen in vapor phase in the presence of a noble metal catalyst. This process serves to produce readily at a low cost 1,1,1,2,3-pentafluoropropane, which is useful and does not cause environmental destruction (i.e. is capable of environmental protection), from an inexpensive starting material in one stage.

## Description

### Industrial fields where the invention can be utilized.

This invention relates to the method of producing 1,1,1,2,3-pentafluoropropane at low cost, which is a hydrofluorocarbon having no fear of destroying ozone layer and may be substituted for chlorofluorocarbons which have been used as working fluids and blowing agents.

### Prior art.

Until now, 1,1,1,2,3-pentafluoropropane is known to be sythesized by the following steps; hexafluoropropene is hydrogenated into hexafluoropropane, then the product is dehydrofluorinated by reacting it with an alkali aqueous solution to produce pentafluoropropene, and finaly the pentafluoropropene is hydrogenated again. However, this reaction is a multistage reaction and complicated. Moreover, hexafluoropropene as a raw material is expensive.

### Objects of the invention.

This invention is aimed at offering a method of easily producing 1,1,1,2,3-pentafluoropropane at low cost, which is useful as a working fluid and so on, and has a property to help preserving the environment.

### The constitution of the invention.

As a result of eagerly studying of efficient methods of 1,1,1,2,3-pentafluoropropane, the inventor found a reduction catalyst by which 1,1,1,2,3-pentafluoropropane can be selectively derived from easily available 1,2,3,3,3-pentafluoro-1-chloropropene at high selectivity in one step, having reached this invention.

That is, this invention relates to a production method of 1,1,1,2,3-pentafluoropropane that is characteristic of hydrogenating 1,2,3,3,3-pentafluoro-1-chloropropene by hydrogen in a gas phase in the presence of a noble metal catalyst.

In the production process of this invention, the above-mentioned noble metal catalyst is desirable to be such a catalyst as one or more than one kind of metals selected from platinum (Pt), palladium (Pd),rhodium (Rh), and ruthenium (Ru) which are carried on a carrier.

In this case, the carrier is active carbon or alumina. And the above-mentioned noble metal catalyst is desirable to be palladium catalyst carried on active carbon made from coconut shell.

In the production process of this invention, the reaction temperature of the above-mentioned hydrogenation is desirable to be from 30 to 500°C.

The invention will be explained in more detail.

To execute the production process of this invention, the raw material 1,2,3,3,3-pentafluoro-1-chloropropene is introduced into a reactor, which is filled with a reduction catalyst shown below (a noble metal catalyst) and is kept at a temperature properly chosen from the range of 30 to 500°C (preferablly of 50 to 300°C), together with hydrogen of 1.5 to 10 times, preferablly 2 to 5 times the raw material, in mole ratio.

As a reduction catalyst, it is better to use such a catalyst as one or two (or more) metals selected from Pd, Pt, Rh, and Ru which are carried on a carrier. As a carrier, a proper carrier selected from active carbon and alumina is used.

As mentioned in examples below, carriers, carried metals, reaction temperatures, and contact times are so closely connected with selectivity and conversion that the reaction temperature and the contact time must be suitably adjusted to a catalyst to be used in order to raise selectivity or conversion. And proper metals are allowed to add to these catalysts to prevent their deterioration.

The most preferable catalyst is Pd catalyst carried on active carbon made from coconut shell. It gives high selectivity and a high yield at a wide range of reaction temperatures.

The raw material 1,2,3,3,3-pentafluoro-1-chloropropene can be easily synthesized as follows. That is, HCFC-21 and pentafluorodichloropropane derived from tetrafluoroethylene are fluorinated in a gas phase by using chromium catalyst to produce 1,1,1,2,2,3-hexafluoro-1-chloropropane, and the product is dehydrofluorinated by reacting it with alkali in water or an alcohol solvent like methanol, ethanol, and isopropanol to be converted to the raw material 1,2,3,3,3-pentafluoro-1-chloropropene.

### The possibility of utilizing the invention in industry.

In the method of this invention, 1,1,1,2,3-pentafluoropropane is produced by hydrogenating 1,2,3,3,3-pentafluoro-1-chloropropene, which is available at a low price, in a gas phase in the presence of a noble metal catalyst. Therefore, 1,1,1,2,3-pentafluoropropane, which doesn't cause environmental disruption (has a property to help preserving the environment) and is useful, can be easily produced in one step and at low cost.

### Embodiments.

The invention will be explained more concretely in the following example.

### Example 1.

10 g of coconut shell active carbon carrying Pd at 0.5% concentration was filled in a Hastelloy-C-made reactor of 20 mmφ and kept at a fixed reaction temperature by using an electric furnace.

H₂ gas was passed into the reactor at a rate of 300 ml/min., and in the stream 1,2,3,3,3-pentafluoro-1-chloropropene was introduced at a rate of 100 ml/min. after vaporizing in a vaporizer.

The products here analyzed by using a gas chromatography (with a polapuc-Q column of 3 m length, kept at 100°C after raising at a rate of 10°C/min.). Using a TCD detector (Thermal Conductivity Detector), each product's yield was obtained after correcting it by its sensitivity.

Though 1,2,3,3,3-pentafluoro-1-chloropropane (indicated as HCFC-235 hereafter) is produced as a by-product, it can be changed to the object compound 1,1,1,2,3-pentafluoropropane (HFC-245) by reacting it again together with 1,2,3,3,3-pentafluoro-1-chloropropene. In the following table-1, the contents of the compounds obtained after the reaction were shown at every reaction temperatures.

**Table - 1**

| Reaction temperature | HFC-245 | HCFC-235 |
|---|---|---|
| 160°C | 76% | 24% |
| 180°C | 96% | 4% |
| 200°C | 99% | 1% |
| 250°C | 100% | - |
| 300°C | 100% | - |

According to this result, it is known that the object compound 1,1,1,2,3-pentafluoropropane can be obtained at a high yield and high selectivity by controlling the reaction temperature.

### Example 2.

The reaction was carried out at the same conditions as those of Example 1 except using alumina catalyst carrying Pd at 0.5% concentration. The mixture obtained fron the reaction consists of the composition shown in the following table-2, indicating that the object product can be obtained at a high yield and high selectivity.

**Table - 2**

| Reaction temperature | HFC-245 | HCFC-235 |
|---|---|---|
| 200°C | 65% | 45% |
| 250°C | 88% | 12% |
| 280°C | 95% | 5% |
| 300°C | 99% | 1% |

### Example 3.

The reaction was carried out at the same conditions as those of Example 1 except using alumina carrying Pt at 0.5% concentration as a catalyst. The mixture obtained from the reaction consists of the composition shown in the following table-3, indicating that the object product can be also obtained at a high yield and high selectivity.

**Table - 3**

| Reaction temperature | HFC-245 | HCFC-235 |
|---|---|---|
| 230°C | 48% | 25% |
| 280°C | 62% | 35% |
| 350°C | 95% | 5% |

### Example 4.

The reaction was carried out at the same conditions as those of Example 1 except using various kinds of catalysts. The mixture obtained from the reaction consists of the composition shown in the following table-4. It is known that the object product can be obtained at a high yield and high selectivity, too.

**Table - 4**

| Catalyst | Reaction temperature | HFC-245 | HCFC-235 |
|---|---|---|---|
| Ru/C (Active carbon) | 250°C | 86% | 14% |
| Ru/Al₂O₃ | 280°C | 79% | 21% |
| Rh/C (Active carbon) | 250°C | 64% | 36% |
| Rh/Al₂O₃ | 280°C | 53% | 47% |

## Claims

1. A method of producing 1,1,1,2,3-pentafluoropropane that is characteristic of hydrogenating 1,2,3,3,3-pentafluoro-1-chloropropene by hydrogen at a gas phase in the presence of a noble metal catalyst.

2. A production method as defined by claim 1, in which a noble metal catalyst is such a catalyst as one or more than one kind of metals selected from platinum, palladium, rhodium, and ruthenium which are carried on a carrier.

3. A production method as defined by claim 2, in which a carrier is active carbon or alumina.

4. A production method as defined by claim 2 or 3, in which a noble metal catalyst is palladium catalyst carried on coconut shell active carbon.

5. A production method as defined by any one of claim 1 - 4, in which the reaction temperature of hydrogenation is within the range from 30 to 500°C.
